# EUROPÄISCHE PATENTSCHRIFT

(11) **EP 2 386 296 B1**
(45) Veröffentlichungstag und Bekanntmachung des Hinweises auf die Patenterteilung: **24.12.2014**
(21) Anmeldenummer: 11003147.3
(22) Anmeldetag: 14.04.2011
(51) Int. Cl.: A61K 9/48, A61K 9/16, A61K 9/50, A61K 31/352

(54) **Neue Darreichungsformen für Cineol**
New application forms for cineol
Nouvelles formulations de cinéole

(30) Priorität: 12.05.2010 DE 102010020425; 21.05.2010 DE 102010022174
(43) Veröffentlichungstag der Anmeldung: 16.11.2011
(73) Patentinhaber: Maria Clementine Martin Klosterfrau Vertriebsgesellschaft mbH, 50670 Köln (DE)
(72) Erfinder: Greve, Harald, Dr., 40545 Düsseldorf (DE); Duchatsch, Walter, Dr., 53721 Siegburg (DE)
(74) Vertreter: Von Rohr Patentanwälte Partnerschaft mbB

(56) Entgegenhaltungen:
- WO-A1-96/40076
- WO-A2-94/28895
- WO-A2-2008/083092
- DE-U1-202009 010 937
- US-A- 4 490 407
- US-A1- 2009 246 279
- DATABASE WPI Week 200877 Thomson Scientific, London, GB; AN 2008-N03211 XP002668644, & CN 101 181 393 A (ZHANG S) 21. Mai 2008 (2008-05-21)
- CHIH PONG CHANG ET AL: "Preparation of alginate complex capsules containing eucalyptus essential oil and its controlled release", COLLOIDS AND SURFACES B: BIOINTERFACES, Bd. 32, Nr. 3, 1. November 2003 (2003-11-01), Seiten 257-262, XP55018183, ISSN: 0927-7765, DOI: 10.1016/j.colsurfb.2003.07.002
- "Feste Arzneiformen; Kapseln" In: BAUER K H; FRÖMMING K-H; FÜHRER; C: "PHARMAZEUTISCHE TECHNOLOGIE", 1993, GEORG THIEME VERLAG, STUTTGART, XP002668732, ISBN: 3-13-692504-1 Bd. 4, Seiten 324-335, * Seite 326; Abbildung 14.41 * * Seite 335, linke Spalte, Absatz 6.3 *

## Beschreibung

Die vorliegende Erfindung betrifft eine in Form einer Kapsel ausgebildete cineolhaltige Darreichungsform für die perorale Applikation und ein Verfahren zu deren Herstellung sowie deren Verwendung, insbesondere im Bereich der Human- und Veterinärmedizin. Des Weiteren betrifft die vorliegende Erfindung ein die erfindungsgemäße cineolhaltige Darreichungsform enthaltendes Arzneimittel oder Medizinprodukt.

1,8-Cineol gehört zu den bicyclischen Epoxy-Monoterpenen, genauer gesagt den Limonenoxiden. Synonyme Bezeichnungen für 1,8-Cineol mit der chemischen Summenformel C₁₀H₁₈O sind Eucalyptol, Limonen-1,8-oxid, 1,8-Epoxy-p-menthan oder 1,3,3-Trimethyl-2-oxabicyclo[2.2.2]octan. Es handelt sich um eine farblose Flüssigkeit mit würzigem, campherähnlichem Geruch mit einem Schmelzpunkt von +1,5 °C und einem Siedepunkt von 176 bis 177 °C, welche in Wasser unlöslich, aber mit den meisten organischen Lösemitteln mischbar ist.

Natürlich kommt 1,8-Cineol als Hauptbestandteil des Eukalyptusöls (Eukalyptusöl enthält bis zu 85 Gew.-% 1,8-Cineol), aber auch in anderen Pflanzen vor, so z. B. in Minze, Heilsalbei, Thymian, Basilikum und im Teebaum. Darüber hinaus ist 1,8-Cineol beispielsweise in Niaouli-, Juniperus-, Piper-, Cannabis-, Kajeput-, Salbeiöl, Myrtenöl und anderen ätherischen Ölen enthalten.

Technisches 1,8-Cineol, welches im Allgemeinen 99,6- bis 99,8-%ig ist, wird im Allgemeinen durch fraktionierte Destillation von Eukalyptusöl gewonnen.

1,8-Cineol wird insbesondere als Expektorans bei Bronchialkatarrhen und anderen Atemwegserkrankungen vorwiegend in der Veterinärmedizin, aber darüber hinaus auch als Aromastoff in der Parfümindustrie angewendet. Darüber hinaus wird 1,8-Cineol in der Zahnmedizin bei der Revision von Wurzelfüllungen verwendet.

In physiologischer Hinsicht wirkt 1,8-Cineol in den oberen und unteren Atemwegen, insbesondere in der Lunge und den Nebenhöhlen, schleimlösend und bakterizid. Außerdem hemmt es bestimmte Neurotransmitter, welche für die Verengung von Bronchien verantwortlich sind. Bei chronisch-obstruktiven Lungenerkrankungen und Asthma bronchiale kann durch Gabe von reinem 1,8-Cineol die Lungenfunktion verbessert werden. Aufgrund seiner kortikosteroidartigen Wirkung wird es im Fall schwerwiegender Atemwegserkrankungen als Substitut zu oder in Komedikation mit Kortikosteroiden unter systemischer Applikation angewendet. 1,8-Cineol kann grundsätzlich sowohl topisch, z. B. inhalativ, oder aber systemisch, insbesondere in Form von Kapseln, angewendet werden.

Als Wirkstoff besitzt 1,8-Cineol also schleimlösende wie entzündungshemmende Wirkungen. Bei systemischer Anwendung wird 1,8-Cineol leicht resorbiert und gelangt über die Blutbahn in den Atmungsorganen zur Wirkung. 1,8-Cineol kann auf diese Weise beispielsweise entzündliche Sekrete sowie zähen Schleim in den Luftwegen verflüssigen und entzündlichen Prozessen in den Atemwegen entgegenwirken. Ein Sekretstau wird so verhindert, das Abhusten erleichtert, die Funktion der für die Reinigung zuständigen Flimmerhärchen in den Bronchien und der Nase unterstützt und somit die Durchlüftung der Atemwege verbessert. Im Bereich der oberen Luftwege schwinden die Behinderung der Nasenatmung bei Schnupfen und die Benommenheit des Kopfes.

Für weitergehende Einzelheiten zu dem Wirkstoff 1,8-Cineol kann beispielsweise verwiesen werden auf Römpp Chemielexikon, Georg Thieme Verlag, Stuttgart/New York, 10. Auflage, Band 1, 1996, Seite 752, Stichwort: "Cineol", sowie die dort referierte Literatur.

Die CN 101 181 393 A beschreibt die Herstellung eines Granulats, welches traditionelle chinesische heilkräftige Komponenten, Zucker, Eukalyptusöl, Ingwertinktur und Ephedrin enthält, wobei das Granulat in Kapseln abgefüllt wird.

Weiterhin betrifft die wissenschaftliche Publikation gemäß Chang et al.: "Preparation of alginate complex capsules containing eucalyptus essential oil and its controlled release Colloid and Surfaces B: Biointerfaces 32 (2003), 257-262 die Herstellung von Kapseln auf Basis eines Alginatkomplexes, welche Eukalyptusöl enthalten sollen.

Die auf die Anmelderin selbst zurückgehende DE 20 2009 010 937 U1 betrifft die Verwendung eines peroralen Monoterpens und eines inhalativen Atemwegstherapeutikums zur prophylaktischen bzw. therapeutischen Behandlung, insbesondere Kombinationstherapie, von Atemwegserkrankungen, wie bronchopulmonalen Erkrankungen.

Weiterhin betrifft der Fachbuchauszug gemäß "Feste Arzneiformen; Kapseln" in: Bauer, K.H., Frömming, K.-H., Führer, C.; "Pharmazeutische Technologie", 1993, Georg Thieme Verlag, Stuttgart, Band 4, Seiten 324-335 pharmazeutisch einsetzbare Kapseln im Allgemeinen.

Darüber hinaus betrifft die US 4 490 407 A ein Verfahren zur Herstellung einer galenischen Formulierung, wobei das Verfahren die Bereitstellung einer flüssigen Mischung mit einem aktiven Inhaltsstoff und einer filmbildenden Substanz umfasst. Die Mischung soll dabei durch Sprühen vertropft und getrocknet werden, um beschichtete Granulate zu erhalten, welche einer anschließenden Lyophilisierung unterzogen werden.

Darüber hinaus betrifft die WO 96/40076 A1 ein Verfahren zur Herstellung von Mikrosphären bzw. -kügelchen, wobei eine Trägersubstanz sowie ein Ausfällmittel zerstäubt und miteinander in Kontakt gebracht werden sollen.

Weiterhin betrifft die WO 2008/083092 A2 ein Verfahren zur Herstellung vielkerniger Mikrokapseln, wobei ein Alkoxysilan in einer Vielphasenemulsion polymerisiert wird, um auf dieser Basis eine Suspension der vielkernigen Mikrokapseln zu bilden.

Zudem betrifft die US 2009/0246279 A1 ein Verfahren zur Herstellung von Partikeln zum Einsatz als Medikament, wobei die Partikel ein hydrophobes Material aufweisen. Insbesondere werden mit Hilfe des beschriebenen Verfahrens hydrophobe Substanzen, wie ätherische Öle, in den Partikeln verkapselt.

In den zu derselben Patentfamilie gehörenden Druckschriften DE 43 19 554 C2, DE 43 19 556 C2 und WO 94/28895 A2 wird eine Kombinationstherapie mit peroral verabreichten Terpenverbindungen, insbesondere 1,8-Cineol oder Menthol, einerseits und ebenfalls systemisch, insbesondere peroral verabreichten Kortikosteroiden andererseits zur entzündungshemmenden Behandlung von systemisch steroidpflichtigem chronischem Asthma bronchiale beschrieben, wobei der Einsatz der peroral verabreichten Terpenverbindungen im Rahmen einer Dauertherapie den Bedarf systemischer Kortikosteroide reduzieren soll. Zum Einsatz kommen bevorzugt magensaftresistente, aber dünndarmlösliche Kapseln mit dem terpenbasierten Wirkstoff.

Für die systemische Anwendung von 1,8-Cineol sind im Handel verschiedene Präparate, insbesondere auf Basis von im Allgemeinen magensaftresistenten, aber dünndarmlöslichen Kapseln, verfügbar, wobei sowohl Monopräparate, welche 1,8-Cineol als einzigen Wirkstoff bzw. in Reinform enthalten, als auch so genannte Kombinationspräparate, welche 1,8-Cineol in komplexer Mischung mit einer Vielzahl weiterer Terpene enthalten, verfügbar bzw. bekannt.

Bei den meisten der im Handel verfügbaren Kapseln mit 1,8-Cineol als Wirkstoff ist im Allgemeinen nicht immer eine optimale Langzeitstabilisierung oder ein optimaler Schutz vor dem Umgebungsmilieu, insbesondere gegenüber Luftoxidation, gegeben. Des Weiteren ist mit den aus dem Stand der Technik bekannten cineolhaltigen Kapselsystemen eine kontrollierte oder retardierte bzw. verzögerte Freisetzung oftmals nicht optimiert bzw. nicht immer möglich. Ebenfalls ist bei den aus dem Stand der Technik bekannten cineolhaltigen Wirkstoffkapseln oftmals auch nicht immer eine optimale Dosierung des Wirkstoffs möglich. Darüber hinaus gelingt mit den aus dem Stand der Technik bekannten cineolhaltigen Kapselsystemen oftmals keine optimale Geschmacks- und/oder Geruchsmaskierung. Auch treten gelegentlich, insbesondere bei Einnahme höherer Dosen, Nebenwirkungen, wie Magen/Darm-Beschwerden, insbesondere Magendruck, Reflux, Übelkeit, Durchfall oder dergleichen, auf.

Daher liegt der vorliegenden Erfindung die Aufgabe zugrunde, eine effiziente, für die perorale Applikation bestimmte Darreichungsform für 1,8-Cineol als Wirkstoff und ein entsprechendes Herstellungsverfahren für diese Darreichungsform bereitzustellen, wodurch die zuvor geschilderten, im Zusammenhang mit dem Stand der Technik auftretenden Nachteile und Nebenwirkungen wenigstens teilweise vermieden oder aber wenigstens zumindest teilweise abgeschwächt werden sollen.

Insbesondere liegt der vorliegenden Erfindung die Aufgabe zugrunde, eine effiziente, für die perorale Applikation bestimmte Darreichungsform für 1,8-Cineol als Wirkstoff bereitzustellen, welche ein verbessertes Freisetzungsprofil und/oder eine verbesserte Langzeitstabilisierung in Bezug auf den Wirkstoff ermöglicht. Insbesondere soll dabei auch der Schutz des Wirkstoffs vor dem Umgebungsmilieu, insbesondere gegenüber Oxidation mit Luftsauerstoff, verbessert werden und zudem eine verbesserte Dosierung und/oder Handhabung des Wirkstoffs, insbesondere auch im Rahmen des Herstellungsverfahrens für die Darreichungsform, gewährleistet werden.

Zur Lösung des zuvor geschilderten Problems schlägt die vorliegende Erfindung - gemäß einem ersten Erfindungsaspekt - eine cineolhaltige Darreichungsform für die perorale Applikation in Kapselform gemäß Anspruch 1; weitere, insbesondere bevorzugte und/oder vorteilhafte Ausgestaltungen dieses Erfindungsaspekts sind Gegenstand der diesbezüglichen Unteransprüche.

Weiterer Gegenstand der vorliegenden Erfindung - gemäß einem zweiten Erfindungsaspekt - ist ein Verfahren zur Herstellung der cineolhaltigen Darreichungsform nach der vorliegenden Erfindung gemäß Anspruch 9; weitere, insbesondere bevorzugte und/oder vorteilhafte Ausgestaltungen dieses Erfindungsaspekts sind Gegenstand der diesbezüglichen Unteransprüche.

Wiederum weiterer Gegenstand der vorliegenden Erfindung - gemäß einem dritten Erfindungsaspekt - ist die Verwendung der cineolhaltigen Darreichungsform nach der vorliegenden Erfindung gemäß Anspruch 11; weitere, insbesondere bevorzugte und/oder vorteilhafte Ausgestaltungen dieses Erfindungsaspekts sind Gegenstand des diesbezüglichen Unteranspruchs.

Schließlich ist weiterer Gegenstand der vorliegenden Erfindung - gemäß einem vierten Erfindungsaspekt - ein Arzneimittel oder Medizinprodukt gemäß Anspruch 14; weitere, insbesondere bevorzugte und/oder vorteilhafte Ausgestaltungen dieses Erfindungsaspekts sind Gegenstand des diesbezüglichen Unteranspruchs.

Es versteht sich von selbst, dass besondere Ausgestaltungen und Ausführungsformen, welche nur im Zusammenhang mit einem Erfindungsaspekt beschrieben sind, auch in Bezug auf die anderen Erfindungsaspekte entsprechend gelten, auch ohne dass dies ausdrücklich beschrieben ist.

Bei allen nachstehend genannten relativen bzw. prozentualen, insbesondere gewichtsbezogenen Mengenangaben ist zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Im Übrigen gilt, dass der Fachmann - insbesondere anwendungsbezogen oder einzelfallbedingt - von den nachfolgend ausgeführten Mengenangaben abweichen kann, ohne dass er den Rahmen der vorliegenden Erfindung verlässt.

Die Anmelderin hat nun überraschenderweise herausgefunden, dass das eingangs geschilderte, der vorliegenden Erfindung zugrundeliegende Problem dadurch gelöst werden kann, dass der Wirkstoff 1,8-Cineol im Rahmen eines Kapseln-in-Kapsel-Systems bereitgestellt wird, wobei der Wirkstoff 1,8-Cineol in inneren Mikrokapseln dieses Kapseln-in-Kapsel-Systems vorhanden ist, so dass das 1,8-Cineol nach systemischer Applikation kontrolliert bzw. retardiert freigesetzt werden kann.

Gegenstand der vorliegenden Erfindung - gemäß einem **ersten** Aspekt der vorliegenden Erfindung - ist somit eine cineolhaltige Darreichungsform für die perorale Applikation in Form einer Kapsel,
wobei die Darreichungsform als Kapseln-in-Kapsel-System ausgebildet ist, wobei das Kapseln-in-Kapsel-System eine äußere Kapsel (Außenkapsel) mit einer äußeren Kapselhülle und eine Mehrzahl in der äußeren Kapsel befindlicher innerer Kapseln (Innenkapseln) aufweist, wobei die inneren Kapseln von der Kapselhülle der äußeren Kapsel vollständig umgeben sind und die inneren Kapseln als Mikrokapseln, welche jeweils den Wirkstoff 1,8-Cineol enthalten, ausgebildet sind, und
wobei die inneren Kapseln 1,8-Cineol zusammen mit mindestens einem mit 1,8-Cineol mischbaren und/oder hierin löslichen, bei 20 °C und Atmosphärendruck flüssigen, physiologisch unbedenklichen Träger (Exzipienten) enthalten, wobei der Träger ausgewählt ist aus der Gruppe von Triglyceriden.

Die vorliegende Erfindung ist mit einer Vielzahl von Vorteilen, Verbesserungen und Besonderheiten verbunden, welche die vorliegende Erfindung gegenüber dem Stand der Technik auszeichnen:
Zum einen ermöglicht die erfindungsgemäße Darreichungsform auf Basis des zuvor beschriebenen Kapseln-in-Kapsel-Systems eine kontrollierte, insbesondere auch retardierte bzw. verzögerte Freisetzung, so dass beispielsweise die Möglichkeit besteht, die für die Tagesdosierung zu applizierende Menge an 1,8-Cineol durch einmalige Verabreichung bereitzustellen, während bei herkömmlichen Cineolwirkstoffkapseln eine mehrmalige Verabreichung über den Tag verteilt erforderlich ist. Zum anderen ermöglicht die erfindungsgemäße Darreichungsform eine effiziente Langzeitstabilisierung des Wirkstoffs 1,8-Cineol, insbesondere auch gegenüber Oxidation beispielsweise durch Luftsauerstoff. Insbesondere wird der Wirkstoff 1,8-Cineol in effizienter Weise vor dem Umgebungsmilieu geschützt, so dass die Lagerzeiten gegenüber herkömmlichen Cineolkapseln signifikant verlängert sind. Darüber hinaus ermöglicht die erfindungsgemäße cineolhaltige Darreichungsform aber auch eine verbesserte Geschmacks- und/oder Geruchsmaskierung des Wirkstoffs. Im Übrigen ist sowohl bei der Herstellung der Kapseln als auch bei ihrer Anwendung die Dosierung und Vermischung in Bezug auf den Wirkstoff optimiert. Weiterhin sind auch unerwünschte Begleiterscheinungen bzw. Nebenwirkungen, insbesondere Magen/Darm-Beschwerden, wie beispielsweise Magendruck, Übersäuerung des Magens, Reflux etc., signifikant reduziert bzw. gänzlich vermieden.

Auf Basis der erfindungsgemäßen cineolhaltigen Darrreichungsform auf der Grundlage des zuvor beschriebenen Kapseln-in-Kapsel-Systems wird also der Wirkstoff 1,8-Cineol bei seiner Lagerung wirksam stabilisiert und vor Umgebungseinflüssen geschützt. Andererseits liegt eine Anwendungsform vor, welche eine kontrollierte Freisetzung bzw. Wirkstoffabgabe im Rahmen eines so genannten *Controlled-Release*-Effektes ermöglicht. Insbesondere lassen sich auf diese Weise Langzeit- oder Depotpräparate bereitstellen, welche den Wirkstoff 1,8-Cineol über einen längeren Zeitraum in genau kontrollierten Mengen abgeben, insbesondere den Wirkstoff gezielt am Wirkungsort freisetzen.

Insbesondere ist es im Rahmen der vorliegenden Erfindung erstmals in Betracht gezogen worden und erstmals gelungen, 1,8-Cineol in vorgenannter Weise einer Mikroverkapselung zuzuführen und auf Basis der so erhaltenen Mikrokapseln das zuvor beschriebene Kapseln-in-Kapsel-System zu konzipieren, welches eine optimale Wirkstoffdosierung und Wirkstoffanwendung von 1,8-Cineol ermöglicht - einhergehend mit den zuvor geschilderten Vorteilen.

Im Stand der Technik dagegen ist die Bereitstellung des Wirkstoffs 1,8-Cineol in mikroverkapselter Form im Rahmen eines Kapseln-in-Kapsel-Systems bislang weder in Betracht gezogen worden, noch konnte eine solche Mikroverkapselung überhaupt realisiert werden. Insbesondere ist es im Stand der Technik bislang nicht gelungen, 1,8-Cineol in effizienter Weise mikrozuverkapseln, was erst im Rahmen der vorliegenden Erfindung erstmals gelungen ist. Dies war in dieser Weise nicht zu erwarten.

Wie zuvor geschildert, wird im Rahmen der vorliegenden Erfindung der Wirkstoff 1,8-Cineol im Rahmen eines Kapseln-in-Kapsel-Systems bereitgestellt, welches eine äußere Kapsel (Außenkapsel) mit einer äußeren Kapselhülle und eine Mehrzahl in der äußeren Kapsel befindlicher innerer Kapseln (Innenkapseln) aufweist, wobei die Innenkapseln von der Außenkapsel vollständig umgeben sind und die Innenkapseln als Mikrokapseln, die jeweils den Wirkstoff 1,8-Cineol enthalten, ausgebildet sind.

Der Begriff der Mikroverkapselung, wie er erfindungsgemäß verwendet wird, bezeichnet insbesondere das Umhüllen, Einbetten etc. des Wirkstoffs 1,8-Cineol mit Hilfe eines geeigneten Kapsel-, Hüll- oder Matrixmaterials, wie nachfolgend noch näher definiert. Bei der Mikroverkapselung werden Mikrokapseln mit definierter Größe und Größenverteilung erzeugt, wie nachfolgend noch geschildert. Grundsätzlich wird dabei, wie nachfolgend gleichermaßen noch geschildert, zwischen Kern/Hülle-Verkapselung einerseits und Matrixverkapselung andererseits unterschieden.

Dementsprechend können im Rahmen der vorliegenden Erfindung die Innenkapseln entweder in Form von Matrixkapseln oder aber in Form von Kern/Hülle-Kapseln vorliegen. Gemäß einer erfindungsgemäß bevorzugten Ausführungsform liegen die Innenkapseln in Form von Matrixkapseln vor. Gleichermaßen besteht im Rahmen der vorliegenden Erfindung die Möglichkeit, Matrixkapseln einerseits und Kern/Hülle-Kapseln andererseits innerhalb eines Kapseln-in-Kapsel-Systems miteinander zu kombinieren.

Von einer Matrixverkapselung spricht man insbesondere dann, wenn der Wirkstoff über die gesamte Mikrokapsel oder Mikrokugel verteilt, bevorzugt in homogener und/oder gleichmäßiger Verteilung, vorliegt, wohingegen bei einer Kern/Hülle-Verkapselung der Wirkstoff im Inneren bzw. im Hohlraum einer Kapselhülle befindlich ist. Matrixkapseln werden insbesondere dann angewandt, wenn eine kontrollierte, insbesondere retardierte und/oder kontinuierliche Freisetzung des verkapselten Wirkstoffs über einen definierten Zeitraum erfolgen soll, wobei durch die geeignete Wahl der Matrixmaterialien und der Kapselgrößen sich unterschiedliche Freisetzungsraten oder/oder unterschiedliche Freisetzungsprofile einstellen lassen, während Kern/Hülle-Verkapselungen insbesondere kurzfristige Freisetzungen oder aber Freisetzungen über einen sehr langen Zeitraum ermöglichen.

Insbesondere weisen die Innenkapseln der erfindungsgemäßen Darreichungsformen eine Kapselhülle oder Kapselmatrix auf, welche den Wirkstoff vollständig einschließt.

Dabei kann grundsätzlich das Kapselhüllen- oder Kapselmatrixmaterial der Innenkapseln in Bezug auf das Kapselhüllenmaterial der Außenkapsel identisch oder verschieden sein. Bevorzugterweise ist das Kapselhüllen- oder Kapselmatrixmaterial der Innenkapseln in Bezug auf das Kapselhüllenmaterial der Außenkapsel verschieden ausgebildet.

Insbesondere kann das Kapselhüllen- oder Kapselmatrixmaterial der Innenkapseln mindestens ein pharmakologisch unbedenkliches Polymer umfassen oder hieraus bestehen. Erfindungsgemäß bevorzugt kommen zu diesem Zweck synthetische, natürliche und/oder naturidentische Polymere und Polymerisate zum Einsatz. Besonders bevorzugt kann das Kapselhüllen- oder Kapselmatrixmaterial der Innenkapseln mindestens ein pharmakologisch unbedenkliches Polymer umfassen oder hieraus bestehen, welches ausgewählt ist aus der Gruppe von (A) Polysacchariden, insbesondere Alginaten, Cellulose und Cellulosederivaten, Chitosanen, Stärke und Stärkederivaten, Agar-Agar, Carrageenen, Pektinen, Galactomannanen, Guaranen, Dextranen, Xanthanen, Glucanen und Gummi arabicum; (B) Proteinen, insbesondere Gelatine und Caseinaten; (C) Polylactiden, insbesondere Homo- und Copolymeren der Milchsäure; (D) Aminoharzen; (E) Poly(meth)acrylaten; (F) Polyharnstoffen; (G) Polyelektrolyten oder Polyelektrolytkomplexen; (H) Wachsen; (I) Paraffinen; (J) Kolloiden und Hydrokolloiden, insbesondere auf Polysaccharid- und/oder Proteinbasis; sowie deren Mischungen und Kombinationen, besonders bevorzugt aus der Gruppe von (A) Polysacchariden, insbesondere Alginaten, Cellulose und Cellulosederivaten, Chitosanen, Stärke und Stärkederivaten, Agar-Agar, Carrageenen, Pektinen, Galactomannanen, Guaranen, Dextranen, Xanthanen, Glucanen und Gummi arabicum; (B) Proteinen, insbesondere Gelatine und Caseinaten; (C) Polylactiden, insbesondere Homo- und Copolymeren der Milchsäure; sowie deren Mischungen und Kombinationen.

Im Allgemeinen können die Innenkapseln der erfindungsgemäßen Darreichungsform mittels Vertropfungsverfahren, Grenzflächenpolykondensationsverfahren, Grenzflächenpolyadditionsverfahren, Lösemittelverdampfungsverfahren, Sprühtrocknungsverfahren oder Phasentrennungsverfahren, bevorzugt mittels Vertropfungsverfahren, hergestellt werden. Erfindungsgemäß bevorzugt sind Innenkapseln, welche mittels Vertropfungsverfahren erhältlich sind; diese Innenkapseln liefern im Rahmen der vorliegenden Erfindung die besten Ergebnisse. Hinsichtlich des Herstellungsverfahrens der Kapseln werden nachfolgend noch detaillierte Ausführungen gemacht werden.

Im Allgemeinen ist der Durchmesser, insbesondere der mittlere Durchmesser, der Innenkapseln mindestens um eine Größenordnung kleiner als der Durchmesser, insbesondere der mittlere Durchmesser, der Außenkapsel ausgebildet.

Im Allgemeinen kann dabei das Verhältnis des Durchmessers, insbesondere des mittleren Durchmessers, der Innenkapseln zum Durchmesser, insbesondere zum mittleren Durchmesser, der Außenkapsel mindestens 1 : 2, insbesondere mindestens 1: 5, vorzugsweise mindestens 1 : 10, besonders bevorzugt mindestens 1 : 15, ganz besonders bevorzugt mindestens 1 : 20, betragen.

Üblicherweise kann das Verhältnis des Durchmessers, insbesondere des mittleren Durchmessers, der Innenkapseln zum Durchmesser, insbesondere zum mittleren Durchmesser, der Außenkapsel bis zu 1 : 100.000, insbesondere bis zu 1 : 50.000, vorzugsweise bis zu 1 : 15.000, besonders bevorzugt bis zu 1 : 10.000, ganz besonders bevorzugt bis zu 1 : 5.000, betragen.

Im Allgemeinen kann das Verhältnis des Durchmessers, insbesondere des mittleren Durchmessers, der Innenkapseln zum Durchmesser, insbesondere zum mittleren Durchmesser, der Außenkapsel im Bereich von 1 : 2 bis 1 : 100.000, insbesondere im Bereich von 1: 5 bis 1: 50.000, vorzugsweise im Bereich von 1 : 10 bis 1 : 15.000, besonders bevorzugt im Bereich von 1 : 15 bis 1 : 10.000, ganz besonders bevorzugt im Bereich von 1 : 20 bis 1: 5.000, variieren.

Der Durchmesser, insbesondere der mittlere Durchmesser, der Innenkapseln kann in weiten Bereichen variieren. Üblicherweise kann der Durchmesser, insbesondere der mittlere Durchmesser, der Innenkapseln im Bereich von 0,1 µm bis 10 mm, insbesondere im Bereich von 1 µm bis 8 mm, vorzugsweise im Bereich von 5 µm bis 7 mm, besonders bevorzugt im Bereich von 10 µm bis 5 mm, ganz besonders bevorzugt im Bereich von 25 µm bis 4 mm, noch mehr bevorzugt im Bereich von 50 µm bis 3 mm, variieren.

Dabei können die Innenkapseln bevorzugt eine monomodale Körnung und/oder Größenverteilung aufweisen.

Auch der Durchmesser, insbesondere der mittlere Durchmesser, der Außenkapsel kann in weiten Bereichen variieren. Üblicherweise kann der Durchmesser, insbesondere der mittlere Durchmesser, der Außenkapsel im Bereich von 50 µm bis 100 mm, insbesondere im Bereich von 100 µm bis 75 mm, vorzugsweise im Bereich von 250 µm bis 60 mm, besonders bevorzugt im Bereich von 500 µm bis 50 mm, ganz besonders bevorzugt im Bereich von 750 µm bis 30 mm, noch mehr bevorzugt im Bereich von 1.000 µm bis 25 mm, variieren.

Wie eingangs geschildert, umfasst das erfindungsgemäße Kapseln-in-Kapsel-System eine Mehrzahl von in einer Außenkapsel befindlichen Mikroinnenkapseln. Insbesondere kann die Außenkapsel mindestens zwei Innenkapseln, insbesondere mindestens fünf Innenkapseln, vorzugsweise mindestens zehn Innenkapseln, besonders bevorzugt mindestens fünfzehn Innenkapseln, ganz besonders bevorzugt mindestens zwanzig Innenkapseln, enthalten. Üblicherweise kann die Außenkapsel bis zu 10.000 Innenkapseln, insbesondere bis zu 5.000 Innenkapseln, vorzugsweise bis zu 1.000 Innenkapseln, besonders bevorzugt bis zu 500 Innenkapseln, ganz besonders bevorzugt bis zu 100 Innenkapseln, enthalten.

Was den Wirkstoff 1,8-Cineol anbelangt, so kann bzw. können die erfindungsgemäße Darreichungsform bzw. die Innenkapseln 1,8-Cineol als alleinigen Wirkstoff enthalten oder aber - gemäß einer alternativen, aber weniger bevorzugten Ausführungsform - den Wirkstoff 1,8-Cineol zumindest mit mindestens einem weiteren Wirkstoff, bevorzugt ausgewählt aus Terpenen, enthalten. Erfindungsgemäß bevorzugt ist es, wenn die erfindungsgemäße Darreichungsform bzw. die Innenkapseln 1,8-Cineol als alleinigen Wirkstoff enthalten.

Erfindungsgemäß enthalten die Innenkapseln 1,8-Cineol zusammen mit mindestens einem mit 1,8-Cineol mischbaren und/oder hierin löslichen, insbesondere bei 20 °C und Atmosphärendruck flüssigen, physiologisch unbedenklichen Träger (Exzipienten). Der Träger bzw. Exzipient ist selbst pharmakologisch nicht wirksam, bildet aber mit dem Wirkstoff 1,8-Cineol eine vorzugsweise homogene Mischung oder Lösung. Auf diese Weise gelingt im Rahmen der vorliegenden Erfindung eine Mikroverkapselung des Wirkstoffs 1,8-Cineol, was ansonsten mit üblichen Mikroverkapselungsmethoden nicht möglich wäre.

Erfindungsgemäß ist der Träger bzw. Exzipient ausgewählt aus der Gruppe von Triglyceriden, besonders bevorzugt mittelkettigen Triglyceriden (*Medium Chain Triglycerides*, MCT), ganz besonders bevorzugt Triglyceriden mit C₆-C₁₂-Fettsäureresten.

Erfindungsgemäß bevorzugt ist es, wenn der Träger bzw. Exzipient in einem 1,8-Cineol/Träger-Mengenverhältnis im Bereich von 1.000 : 1 bis 1 : 1.000, insbesondere 100 : 1 bis 1 : 100, vorzugsweise 50 : 1 bis 1 : 50, besonders bevorzugt 10 : 1 bis 1 : 10, ganz besonders bevorzugt 5 : 1 bis 1 : 2, noch mehr bevorzugt 3 : 1 bis 1 : 1, eingesetzt wird; auf diese Weise gelingt eine besonders gute Mikroverkapselung des Wirkstoffs 1,8-Cineol.

Der Begriff der Öle, wie er erfindungsgemäß für die vorstehenden Träger bzw. Exzipienten verwendet wird, ist eine Sammelbezeichnung für Flüssigkeiten, welche sich nicht mit Wasser mischen lassen. Der Begriff der fetten Öle, wie er in diesem Zusammenhang erfindungsgemäß verwendet wird, bezeichnet speziell Fette, d. h. Gemische von Fettsäuretriglyceriden, welche bei Raumtemperatur (insbesondere 20 °C) und Atmosphärendruck flüssig sind; insbesondere bezeichnet dieser Begriff Ester des dreiwertigen Alkohols Glycerin (Propan-1,2-3-triol) mit drei, meist verschiedenen, überwiegend geradzahligen und unverzweigten aliphatischen Monocarbonsäuren, den so genannten Fettsäuren. Verbindungen dieser Art werden auch Triglyceride genannt (nach IUPAC-Empfehlung: Triacylglycerine). Triglyceride, synonym auch als Glycerol-Triester bezeichnet, sind also dreifache Ester des dreiwertigen Alkohols Glycerin mit drei Säuremolekülen, wobei die Vorsilbe "*tri*" auf drei Acyl-Säurereste, die mit Glycerin verestert sind, verweist.

Speziell mittelkettige Triglyceride, wie sie erfindungsgemäß bevorzugt als Träger bzw. Exzipient für den Wirkstoff 1,8-Cineol zum Einsatz kommen, sind insbesondere halbsynthetische neutrale Glycerinester von gesättigten, im Allgemeinen unverzweigten Monocarbonsäuren mittlerer Kettenlänge (d. h. C₆-C₁₂). Insbesondere bezeichnet der Begriff der mittelkettigen Triglyceride Gemische von Triglyceriden gesättigter Fettsäuren, hauptsächlich Caprylsäure (Octansäure) und Caprinsäure (Decansäure). Mittelkettige Triglyceride können im Allgemeinen aus Öl hergestellt werden, das aus dem festen und getrockneten Teil des Endosperms von *Cocos nucifera* L. und/oder aus dem getrockneten Endosperm von *Elaeis guineenses* Jacq. extrahiert wird. Für weitergehende Einzelheiten zu dem Begriff der mittelkettigen Triglyceride kann beispielsweise verwiesen werden auf die Monographie Ph. Eur. 6., Ausgabe, Grundwerk 2008, Seiten 4224 bis 4226, sowie auf die Zeitschrift für Ernährungswissenschaft, Band 13, Heft 1/2, 1973, Seiten 6 ff., D. Sailer et al. "Mittelkettige Triglyceride - Klinische Physiologie und Anwendung*"*).

Der Wirkstoffgehalt (d. h. der 1,8-Cineolgehalt) der erfindungsgemäßen Darreichungsform kann gleichermaßen in weiten Grenzen variieren: Üblicherweise enthält die erfindungsgemäße Darreichungsform den Wirkstoff 1,8-Cineol in absoluten Mengen von 10 bis 1.000 mg, insbesondere 25 bis 750 mg, vorzugsweise 50 bis 500 mg, besonders bevorzugt 75 bis 300 mg. Im Allgemeinen enthält die erfindungsgemäße Darreichungsform den Wirkstoff 1,8-Cineol in relativen Mengen von 0,01 bis 99 Gew.-%, insbesondere 0,1 bis 95 Gew.-%, vorzugsweise 1 bis 90 Gew.-%, besonders bevorzugt 5 bis 80 Gew.-%.

Im Rahmen der vorliegenden Erfindung liegt die erfindungsgemäße Darreichungsform im Allgemeinen in magensaftresistenter, dünndarmlöslicher Form vor, d. h. die erfindungsgemäße Darreichungsform ist üblicherweise magensaftresistent, aber dünndarmlöslich ausgebildet. Auf diese Weise wird erreicht, dass der Wirkstoff 1,8-Cineol bei systemischer Applikation erst am eigentlichen Resorptionsort, d. h. dem Dünndarm, freigesetzt wird. Zu diesem Zweck kann bzw. können die Außenkapsel und/oder die Innenkapseln in magensaftresistenter, dünndarmlöslicher Form vorliegen, bevorzugt mit einer magensaftresistenten, dünndarmlöslichen Beschichtung oder Hülle versehen sein. Derartige magensaftresistente, aber dünndarmlösliche Beschichtungs- oder Hüllmaterialien sind dem Fachmann als solches bekannt: Zu diesem Zweck können beispielsweise (Meth-)Acrylsäure-Polymere, insbesondere Methacrylsäure/Ethylacrylat-Copolymere (z. B. Eudragit^{®}), eingesetzt werden; für weitergehende Einzelheiten zu für diesen Zweck eingesetzten Methacrylsäure/Ethylacrylat-Copolymeren kann beispielsweise auf die Monographie Ph. Eur., 6. Ausgabe, Grundwerk 2008, Seiten 3215 bis 3217, verwiesen werden. Alternativ, gemäß einer erfindungsgemäß bevorzugten Ausführungsform, kann zu diesem Zweck auch ein Gemisch aus Ölsäure, Stearinsäure und Ethylcellulose als magensaftresistente, dünndarmlösliche Beschichtung bzw. Überzug eingesetzt werden.

Weitere vorteilhafte Eigenschaften, Aspekte und Merkmale der vorliegenden Erfindung ergeben sich aus der folgenden Beschreibung eines in den Figuren dargestellten Ausführungsbeispiels. Es zeigt:
- Fig.: eine schematische Schnittdarstellung durch eine erfindungsgemäße cineolhaltige Darreichungsform gemäß einer Ausführungsform der vorliegenden Erfindung.

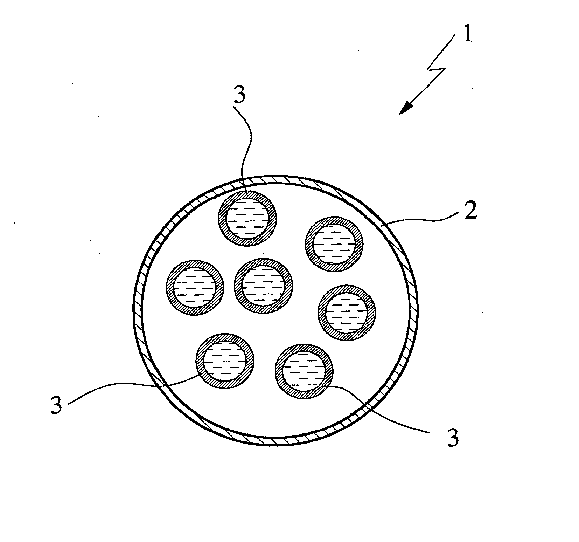

Die einzige Figur zeigt eine erfindungsgemäße, für die perorale Applikation bestimmte, in Form einer Kapsel ausgebildete cineolhaltige Darreichungsform 1 gemäß einer Ausführungsform der vorliegenden Erfindung. Wie sich aus der Figurendarstellung ergibt, ist die Darreichungsform 1 als Kapseln-in-Kapsel-System ausgebildet ist, wobei das Kapseln-in-Kapsel-System eine äußere Kapsel (Außenkapsel) 2 mit einer äußeren Kapselhülle und eine Mehrzahl in der äußeren Kapsel befindlicher innerer Kapseln (Innenkapseln) 3 aufweist, wobei die inneren Kapseln 3 von der Kapselhülle der äußeren Kapsel 2 vollständig umgeben sind und die inneren Kapseln 3 als Mikrokapseln, welche jeweils den Wirkstoff 1,8-Cineol enthalten, ausgebildet sind.

Für weitergehende Einzelheiten zu der in der Figurendarstellung dargestellten Ausführungsform kann zur Vermeidung unnötiger Wiederholungen auf die obigen Ausführungen verwiesen werden, welche in Bezug auf die Figurendarstellung entsprechend gelten.

Weiterer Gegenstand der vorliegenden Erfindung - gemäß einem **zweiten** Aspekt der vorliegenden Erfindung - ist ein Verfahren zur Herstellung einer cineolhaltigen Darreichungsform, wie zuvor beschrieben,
wobei zunächst Mikrokapseln hergestellt werden, welche jeweils den Wirkstoff 1,8-Cineol zusammen mit mindestens einem mit 1,8-Cineol mischbaren und/oder hierin löslichen, bei 20 °C und Atmosphärendruck flüssigen, physiologisch unbedenklichen Träger (Exzipienten) enthalten, wobei der Träger ausgewählt ist aus der Gruppe von Triglyceriden, und
wobei nachfolgend eine Mehrzahl der zuvor hergestellten Mikrokapseln von einer äußeren Kapselhülle vollständig umgeben und zu einem Kapseln-in-Kapsel-System mit den Mikrokapseln als innere Kapseln (Innenkapseln) zusammengeführt und/oder zusammengefasst wird.

Wie zuvor beschrieben, kann die Herstellung der Mikrokapseln bzw. Innenkapseln mittels Vertropfungsverfahren, Grenzflächenpolykondensationsverfahren, Grenzflächenpolyadditionsverfahren, Lösemittelverdampfungsverfahren, Sprühtrocknungsverfahren oder Phasentrennungsverfahren erfolgen. In erfindungsgemäß bevorzugter Weise wird für die Herstellung der Mikrokapseln bzw. Innenkapseln ein Vertropfungsverfahren angewendet. Die vorgenannten Verfahren sind dem Fachmann als solches grundsätzlich bekannt, bislang aber weder für den Wirkstoff 1,8-Cineol noch im Zusammenhang mit dem erfindungsgemäßen Gesamtverfahren in Kombination mit den übrigen Verfahrensschritten angewendet worden.

Bislang ist im Stand der Technik die Herstellung eines 1,8-Cineol enthaltenden Kapseln-in-Kapsel-Sytems mit 1,8-Cineol enthaltenden Mirkokapseln als inneren Kapseln (Innenkapseln) nicht gelungen, da 1,8-Cineol sich nicht ohne weiteres homogen zu entsprechenden Mikrokapseln verarbeiten lässt, insbesondere aufgrund seines relativ hohen Schmelzpunkts von +1,5 °C.

Die Anmelderin hat aber nun in völlig überraschender und unerwarteter Weise herausgefunden, dass die Herstellung von 1,8-Cineol enthaltenden Mikrokapseln, insbesondere als innere Kapseln (Innenkapseln) eines Kapseln-in-Kapsel-Systems gelingt, wenn die Herstellung der Mikrokapseln in Gegenwart eines wie zuvor beschriebenen Trägers bzw. Exzipienten für das 1,8-Cineol durchgeführt wird, d. h. die Mikroverkapselung eines Gemisches bzw. einer Lösung von 1,8-Cineol einerseits und einem Träger bzw. Exzipienten der vorgenannten Art andererseits durchgeführt wird.

Die Herstellung der 1,8-Cineol enthaltenden Mikrokapseln bzw. inneren Kapseln (Innenkapseln) erfolgt im Rahmen der vorliegenden Erfindung dabei bevorzugt mittels eines Vertropfungsverfahrens, wobei ein mikrokapselbildendes Ausgangsmaterial (z. B. Alginate) in Gegenwart von 1,8-Cineol und gegebenenfalls mindestens eines mit 1,8-Cineol mischbaren und/oder hierin löslichen, bei 20 °C und Atmosphärendruck flüssigen, physiologisch unbedenklichen Trägers (Exzipienten), insbesondere wie zuvor beschrieben, vertropft und zu 1,8-Cineol enthaltenden Mikrokapseln verfestigt wird. Für weitergehende diesbezügliche Einzelheiten in Bezug auf den Träger bzw. Exzipienten kann zur Vermeidung unnötiger Wiederholungen auf die obigen Ausführungen verwiesen werden.

Im Rahmen des erfindungsgemäßen Herstellungsverfahrens kann bei dem Verfahrensschritt der Mikroverkapselung des Wirkstoffs 1,8-Cineol insbesondere wie folgt vorgegangen werden:

Der Wirkstoff 1,8-Cineol wird mit dem zuvor geschilderten Träger bzw. Exzipienten in Kontakt gebracht und innig bzw. homogen vermischt. Die resultierende Mischung bzw. Lösung aus 1,8-Cineol und Träger bzw. Exzipient wird dann nachfolgend einer Mikroverkapselung unterzogen.

Zu diesem Zweck kann die mikrozuverkapselnde Flüssigkeit aus 1,8-Cineol/Träger bzw. Exzipient zusammen mit dem kapselbildenden Material (z. B. Alginaten) unter Druck einem geeigneten Düsenkopf zugeführt und mittels dieses Düsenkopfes zu Mikrokugeln vertropft werden, was beispielsweise durch geeignete Schwingung erfolgen kann, infolge derer der aus dem Düsenkopf austretende Flüssigkeitsstrahl in einzelne Segmente bzw. Kügelchen eingeschnürt und zerteilt wird, welche nachfolgend, insbesondere infolge der Oberflächenspannung, Kugelgestalt annehmen und dann in einem geeigneten Medium (z. B. einer geeignete Ionenlösung) verfestigt werden.

Ein für diese Zwecke einsetzbares Mikroverkapselungsverfahren ist beispielsweise in der FR 2 645 439 A1 bzw. in der zu derselben Patentfamilie gehörigen EP 0 391 803 B1 beschrieben, deren jeweiliger Offenbarungsgehalt hiermit durch Bezugnahme eingeschlossen ist: Gemäß diesem Verfahren werden alginatbasierte Mikrokapseln durch ein Vertropfungsverfahren bereitgestellt, welches sich im Fall der vorliegenden Erfindung speziell auf die Mikroverkapselung der zuvor beschriebenen 1,8-Cineol/Träger-Mischung anwenden lässt. Dabei werden die mit Wirkstoff 1,8-Cineol beladenen Alginattröpfchen in einer geeigneten Ionenflüssigkeit (z. B. Calciumchloridlösung) verfestigt.

Erfindungsgemäß zur Herstellung der Mikrokapseln bzw. Innenkapseln gleichermaßen geeignet ist das in der WO 93/02785 A1 und DE 41 25 133 A1 beschriebene Vertropfungsverfahren, deren jeweiliger Offenbarungsgehalt hiermit durch Bezugnahme eingeschlossen ist. Gemäß diesem Verfahren werden alginatbasierte Mikrokapseln hergestellt, wobei zu diesem Zweck alginatbasierte, die bereits beschriebene 1,8-Cineol/Träger-Mischung enthaltende Alginatkugeln aus Tropfen aus von einer Düse abgegebener Alginatlösung durch Verfestigung der Tropfen durch Eintropfen dieser in eine Ionenlösung und gegebenenfalls anschließendes Waschen von aus der Ionenlösung entnommenen Alginatkugeln hergestellt werden, wobei die Alginatlösung durch Schwingungsanregung zertropft werden kann und die Tropfen bis zur gewünschten Verfestigung in der Ionenlösung im Wesentlichen frei beweglich sind.

Der Offenbarungsgehalt aller vorgenannter, im Zusammenhang mit der Mikroverkapselung erwähnter Druckschriften ist hiermit durch Bezugnahme eingeschlossen, so dass auf weitergehende Einzelheiten in Bezug auf die Herstellung der im Rahmen des erfindungsgemäßen Verfahrens zu erzeugenden Mikrokapseln auf die zuvor genannten Druckschriften verwiesen werden kann.

Das Verfahren der Mikroverkapselung wird also in für den Fachmann an sich bekannter Weise durchgeführt, jedoch insbesondere mit der Maßgabe, dass für die Verkapselung eine zuvor beschriebene Lösung bzw. Mischung aus 1,8-Cineol und speziellen Exzipienten bzw. Träger zum Einsatz kommt.

An die Verfahrensschritte der Herstellung der 1,8-Cineol enthaltenden Wirkstoffmikrokapseln schließt sich dann die Aufbringung der Außenhülle bzw. Außenschicht jeweils auf bzw. um eine Mehrzahl der auf diese Weise hergestellten Mikrokapseln an. Hierbei handelt es sich um einen für den Fachmann an sich bekannten Verfahrensschritt, so dass sich weitergehende diesbezügliche Einzelheiten erübrigen.

In Bezug auf weitergehende Einzelheiten zu dem erfindungsgemäßen Verfahren kann zur Vermeidung unnötiger Wiederholungen auf die obigen Ausführungen zu der erfindungsgemäßen Darreichungsform verwiesen werden, welche bezüglich des erfindungsgemäßen Verfahrens entsprechend gelten.

Weiterer Gegenstand der vorliegenden Erfindung - gemäß einem **dritten** Aspekt der vorliegenden Erfindung - ist eine wie zuvor beschriebene cineolhaltige Darreichungsform nach der vorliegenden Erfindung zur Verwendung in der Humanmedizin und in der Veterinärmedizin.

Gegenstand der vorliegenden Erfindung ist somit eine erfindungsgemäße cineolhaltige Darreichungsform zur prophylaktischen und/oder therapeutischen Behandlung von entzündlichen, infektexazerbierten oder allergischen Erkrankungen des menschlichen oder tierischen Körpers. Gleichermaßen kann die erfindungsgemäße Darreichungsform zur Herstellung einer Arzneimittels zur prophylaktischen und/oder therapeutischen Behandlung von entzündlichen, infektexazerbierten oder allergischen Erkrankungen des menschlichen oder tierischen Körpers verwendet werden.

Gleichermaßen kann beispielsweise die erfindungsgemäße cineolhaltige Darreichungsform zur prophylaktischen und/oder therapeutischen Behandlung von Autoimmunerkrankungen des menschlichen oder tierischen Körpers angewendet werden.

Weiterhin kann die erfindungsgemäße cineolhaltige Darreichungsform zur prophylaktischen und/oder therapeutischen Behandlung von Erkältungserkrankungen und grippalen Infekten und hiermit zusammenhängenden Erkrankungen und Infekten, insbesondere Infekten der oberen und unteren Atemwege, wie insbesondere Rhinitis, Sinusitis und bronchopulmonalen Erkrankungen, angewendet werden.

Weiterhin kann die erfindungsgemäße cineolhaltige Darreichungsform zur prophylaktischen und/oder therapeutischen Behandlung von Atemwegserkrankungen, insbesondere von bronchopulmonalen Erkrankungen, insbesondere Bronchitis, Asthma bronchiale und chronischen obstruktiven Lungenerkrankungen (COPD), angewendet werden. In diesem Zusammenhang ist auch eine Komedikation mit anderen Therapeutika bzw. Arzneimitteln, insbesondere Kortikosteroiden, möglich.

Gleichermaßen kann die erfindungsgemäße cineolhaltige Darreichungsform auch zur prophylaktischen und/oder therapeutischen Behandlung von entzündlichen Erkrankungen der Gallenwege, insbesondere Cholezystitis und Cholangitis, angewendet werden.

Des Weiteren kann die cineolhaltige Darreichungsform nach der vorliegenden Erfindung auch zur prophylaktischen und/oder therapeutischen Behandlung von entzündlichen Erkrankungen der ableitenden Harnwege, insbesondere Glomerulonephritis, Pyelonephritis, Zystitis und Uritritis, Anwendung finden.

Ebenfalls kann die erfindungsgemäße cineolhaltige Darreichungsform gleichermaßen Anwendung finden zur prophylaktischen und/oder therapeutischen Behandlung von entzündlichen Erkrankungen des Darms, insbesondere Morbus Crohn und Colitis ulcerosa.

Gleichermaßen kann die erfindungsgemäße Darreichungsform auch Anwendung finden zur prophylaktischen und/oder therapeutischen Behandlung von entzündlichen oder allergischen Hauterkrankungen, insbesondere Ekzemen und Dermatitis.

Des Weiteren kann die erfindungsgemäße Darreichungsform auch Anwendung finden zur prophylaktischen und/oder therapeutischen Behandlung von rheumatoiden Erkrankungen (d. h. Rheuma bzw. Rheumatismus sowie sämtliche Erkrankungen des rheumatischen Formenkreises).

Schließlich kann die erfindungsgemäße Darreichungsform auch Anwendung finden zur prophylaktischen und/oder therapeutischen Behandlung von systemisch kortikosteroidpflichtigen Erkrankungen (d. h. sämtlichen Erkrankungen, welche mit systemisch verabreichten Kortikosteroiden therapiert werden müssen.). Dies lässt sich durch das kortikosteroidartige pharmakologische Wirkprofil von 1,8-Cineol erklären.

Bei allen vorstehenden Verwendungen ist eine Komedikation mit mindestens einem weiteren Therapeutikum und/oder Arzneimittel möglich.

Im Rahmen der erfindungsgemäßen Verwendung wird die cineolhaltige Darreichungsform nach der vorliegenden Erfindung üblicherweise in solchen Mengen verabreicht, dass das 1,8-Cineol in Tagesdosen von 50 bis 3.000 mg/diem, insbesondere 100 bis 2.000 mg/diem, vorzugsweise 200 bis 1.000 mg/diem, verabreicht wird und/oder dass das 1,8-Cineol zur Verabreichung in einer Tagesdosis von 50 bis 3.000 mg/diem, insbesondere 100 bis 2.000 mg/diem, vorzugsweise 200 bis 1.000 mg/diem, hergerichtet ist.

Für weitergehende diesbezügliche Einzelheiten zu der erfindungsgemäßen Verwendung kann zur Vermeidung unnötiger Wiederholungen auf die obigen Ausführungen zu der erfindungsgemäßen Darreichungsform und zu dem erfindungsgemäßen Herstellungsverfahren verwiesen werden, welche hinsichtlich der erfindungsgemäßen Verwendung entsprechend gelten.

Schließlich ist Gegenstand der vorliegenden Erfindung - gemäß einem **vierten** Aspekt der vorliegenden Erfindung - ein Arzneimittel oder Medizinprodukt, welches die zuvor beschriebene cineolhaltige Darreichungsform nach der vorliegenden Erfindung enthält.

Im Rahmen des erfindungsgemäßen Arzneimittels oder Medizinprodukts ist das Arzneimittel oder Medizinprodukt insbesondere für eine Verabreichung von 1,8-Cineol in einer Tagesdosis von 50 bis 3.000 mg/diem, insbesondere 100 bis 2.000 mg/diem, vorzugsweise 200 bis 1.000 mg/diem, hergerichtet.

Für weitergehende diesbezügliche Einzelheiten zu dem erfindungsgemäßen Arzneimittel oder Medizinprodukt kann zur Vermeidung unnötiger Wiederholungen auf die obigen Ausführungen zu der erfindungsgemäßen Darreichungsform, dem erfindungsgemäßen Herstellungsverfahren und der erfindungsgemäßen Verwendung Bezug genommen werden, welche hinsichtlich des erfindungsgemäßen Arzneimittels oder Medizinprodukts entsprechend gelten.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne dass er dabei den Rahmen der vorliegenden Erfindung verlässt.

Die vorliegende Erfindung wird anhand der folgenden Ausführungsbeispiele veranschaulicht, welche die vorliegende Erfindung jedoch keinesfalls beschränken.

### Ausführungsbeispiele:

### Herstellung einer erfindungsgemäßen Darreichungsform

Eine erfindungsgemäße Darreichungsform auf Basis eines Kapseln-in-Kapsel-Systems wird wie folgt hergestellt:
Zunächst wird eine homogene Mischung auf Basis von 1,8-Cineol einerseits und mittelkettigen Triglyceriden andererseits in einem Gewichtsverhältnis von etwa 2 : 1 hergestellt.

Auf Basis dieser 1,8-Cineol/Triglyceride-Mischung werden nachfolgend entsprechend dem Verfahren und der Vorrichtung gemäß FR 2 645 436 A1 alginatbasierte Matrixmikrokapseln, welche den Wirkstoff 1,8-Cineol enthalten, mittels des dort beschriebenen Vertropfungsverfahrens hergestellt.

Zu diesem Zweck wird eine wässrige Natriumalginatlösung, welche die zuvor beschriebene 1,8-Cineol/Triglyceride-Mischung enthält, gegebenenfalls zusammen mit üblichen Additiven hergestellt und nachfolgend diese, 1,8-Cineol enthaltende wässrige Natriumalginatlösung in an sich bekannter Weise in eine vernetzende, Calciumchlorid enthaltende Lösung, welche darüber hinaus übliche Additive (z. B. Tenside etc.) enthalten kann, vertropft.

Es resultieren 1,8-Cineol als Wirkstoff enthaltende Matrixmikrokapseln mit Alginat als Matrixmaterial. Die so erhaltenen Mikrokapseln werden nachfolgend aufgereinigt und gegebenenfalls isoliert.

Alternativ zur vorbeschriebenen Ausführungsweise können entsprechend der FR 2 645 439 A1 auch zunächst reine alginatbasierte Matrixmikrokapseln (d.h. Matrixmikrokapseln ohne den Wirkstoff 1,8-Cineol hergestellt werden), welche nachfolgend in eine Lösung auf Basis der zuvor beschriebenen 1,8-Cineol/Triglyceride-Mischung eingebracht und auf diese Weise hiermit beladen werden.

Die auf diese Weise erhaltenen, mit dem Wirkstoff 1,8-Cineol beladenen Mikromatrixkapseln werden nachfolgend in an sich bekannter Weise zu einem erfindungsgemäßen Kapseln-in-Kapsel-System weiterverarbeitet, indem jeweils eine Mehrzahl der zuvor beschriebenen Mikrokapseln mit einer Außenhülle umgeben wird.

Die Außenhülle wird abschließend mit einem magensaftresistenten, aber dünndarmlöslichen Überzug versehen. Zu diesem Zweck können beispielsweise (Meth-)Acrylsäure-Polymere, insbesondere Methacrylsäure/Ethylacrylat-Copolymere (z. B. Eudragit^{®}), eingesetzt werden. Alternativ hierzu kann zu diesem Zweck aber auch ein Gemisch aus Ölsäure, Stearinsäure und Ethylcellulose eingesetzt werden.

### Klinische Anwendungsbeobachtungen und Stabilitätsuntersuchungen

Die zuvor beschriebene, 1,8-Cineol als Wirkstoff enthaltende Darreichungsform nach der vorliegenden Erfindung auf Basis eines Kapseln-in-Kapsel-Systems wird im Rahmen der Behandlung von Asthma bronchiale in Komedikation zu Kortikosteroiden eingesetzt.

Als Vergleich dienen handelsübliche 1,8-Cineol enthaltende Kapseln, welche jeweils 200 mg 1,8-Cineol pro Kapsel enthalten, wobei die handelsüblichen Kapseln einen flüssigen, 1,8-Cineol enthaltenen Kern und eine äußere, magensaftresistente, aber dünndarmlösliche Hüllschicht aufweisen.

Im Rahmen der klinischen Anwendungsbeobachtungen werden jeweils 15 Probanden mit handelsüblichen 1,8-Cineol enthaltenen Kapseln mit dreimaliger Gabe von 200 mg/diem einerseits und mit der erfindungsgemäßen Darreichungsform mit einmaliger Gabe von einmal 500 mg/diem andererseits behandelt. Bereits nach 4tägiger Therapie mit 1,8-Cineol wird die Lungenfunktion (Anstieg der FeV₁, Abnahme des Atemwegwiderstandes, Raw) in gleicher Weise verbessert, und in allen Fällen kann in Folge einer zunehmenden Stabilisierung des Bronchialasthmas der tägliche Kortikosteroidbedarf um ca. 50 % bis 60 % reduziert werden. Im Rahmen der erfindungsgemäßen Darreichungsform bewirkt die nur einmalige tägliche Verabreichung mit um 100 mg/diem reduzierter Wirkdosis denselben Effekt wie die dreimalige Gabe von jeweils 200 mg/diem mit handelsüblichen Kapseln, welche den Wirkstoff nicht mit Mikroverkapselung enthalten.

Die Dauertherapie mit 1,8-Cineol wird bei dem erfindungsgemäßen Kapseln-in-Kapsel-System deutlich besser vertragen, insbesondere sind dort Nebenwirkungen, wie Magen/Darm-Beschwerden, insbesondere Magendruck, Magenübersäuerung und Reflux, signifikant reduziert.

Auch die Lagerstabilität unter standardisierten Lagerungsbedingungen ist im Fall der erfindungsgemäßen Darreichungsform gegenüber handelsüblichen 1,8-Cineol enthaltenden Kapseln um mehr als 70 % verbessert und somit optimiert.

Im Rahmen des erfindungsgemäßen Herstellungsverfahrens ist zudem die Einstellung der Wirkstoffmenge pro Kapsel gegenüber der Herstellung herkömmlicher 1,8-Cineol enthaltender Kapseln deutlich verbessert und folglich optimiert.

Im Ergebnis führt die erfindungsgemäße Darreichungsform auf Basis des 1,8-Cineol enthaltenen Kapseln-in-Kapsel-Systems zu einer Verbesserung und Optimierung im Hinblick auf die Herstellbarkeit sowie die Anwendungseigenschaften von 1,8-Cineol enthaltenden Kapselsystemen.

### Weitere klinische Anwendungsbeobachtungen

Die zuvor beschriebene, 1,8-Cineol als Wirkstoff enthaltende Darreichungsform nach der vorliegenden Erfindung auf Basis eines Kapseln-in-Kapsel-Systems wird im Rahmen der Behandlung von Morbus Crohn und Colitis ulcerosa in Komedikation zu Kortikosteroiden eingesetzt.

Als Vergleich dienen wiederum handelsübliche 1,8-Cineol enthaltende Kapseln, welche jeweils 200 mg 1,8-Cineol pro Kapsel enthalten, wobei die handelsüblichen Kapseln einen flüssigen, 1,8-Cineol enthaltenen Kern und eine äußere, magensaftresistente, aber dünndarmlösliche Hüllschicht aufweisen.

Im Rahmen der klinischen Anwendungsbeobachtungen werden jeweils 12 Probanden mit handelsüblichen 1,8-Cineol enthaltenen Kapseln mit dreimaliger Gabe von 200 mg/diem einerseits und mit der erfindungsgemäßen Darreichungsform mit einmaliger Gabe von einmal 500 mg/diem andererseits behandelt.

Aufgrund der entzündlichen Komponente lassen sich im Rahmen der klinischen Anwendung in Analogie zur Therapie von Asthma bronchiale auch ein Morbus Crohn und eine Colitis ulcerosa behandeln mit dem gleichen Therapieregime behandeln.

Auch hier kann mit der erfindungsgemäßen Darreichungsform mit der geringeren Dosierung der gleiche positive klinische Effekt bewirkt werden, welcher dadurch zum Ausdruck kommt, dass die Stuhlfrequenz um fast die Hälfte reduziert wird. Ebenfalls kann auch hier die Dosis der systemischen Kortikosteroide um ca. 60 % bis 70 % reduziert werden. Im Rahmen der erfindungsgemäßen Darreichungsform bewirkt die nur einmalige tägliche Verabreichung mit um 100 mg/diem reduzierter Wirkdosis denselben Effekt wie die dreimalige Gabe von jeweils 200 mg/diem mit handelsüblichen Kapseln, welche den Wirkstoff nicht mit Mikroverkapselung enthalten.

Vergleichbare Ergebnisse werden auch bei der Therapie systemisch kortikosteroidpflichtiger rheumatischer bzw. rheumatoider Erkrankungen erhalten: Auch hier die Dosis der systemischen Kortikosteroide um ca. 50 % bis 70 % reduziert werden, wobei auch diesbezüglich im Rahmen der erfindungsgemäßen Darreichungsform die nur einmalige tägliche Verabreichung mit um 100 mg/diem reduzierter Wirkdosis denselben Effekt wie die dreimalige Gabe von jeweils 200 mg/diem mit handelsüblichen Kapseln, welche den Wirkstoff nicht mit Mikroverkapselung enthalten, bewirkt.

## Patentansprüche

1. Cineolhaltige Darreichungsform für die perorale Applikation in Form einer Kapsel,
wobei die Darreichungsform als Kapseln-in-Kapsel-System ausgebildet ist, wobei das Kapseln-in-Kapsel-System eine äußere Kapsel (Außenkapsel) mit einer äußeren Kapselhülle und eine Mehrzahl in der äußeren Kapsel befindlicher innerer Kapseln (Innenkapseln) aufweist, wobei die inneren Kapseln von der Kapselhülle der äußeren Kapsel vollständig umgeben sind und die inneren Kapseln als Mikrokapseln, welche jeweils den Wirkstoff 1,8-Cineol enthalten, ausgebildet sind, und
wobei die inneren Kapseln 1,8-Cineol zusammen mit mindestens einem mit 1,8-Cineol mischbaren und/oder hierin löslichen, bei 20 °C und Atmosphärendruck flüssigen, physiologisch unbedenklichen Träger (Exzipienten) enthalten, wobei der Träger ausgewählt ist aus der Gruppe von Triglyceriden.

2. Darreichungsform nach Anspruch 1, **dadurch gekennzeichnet,**
**dass** die Innenkapseln in Form von Kern/Hülle-Kapseln vorliegen und/oder **dass** die Innenkapseln eine Kapselhülle oder Kapselmatrix, welche den Wirkstoff vollständig einschließt, aufweisen und/oder
**dass** das Kapselhüllen- oder Kapselmatrixmaterial der Innenkapseln in Bezug auf das Kapselhüllenmaterial der Außenkapsel identisch oder verschieden sein kann und bevorzugt verschieden ist.

3. Darreichungsform nach einem der vorangehenden Ansprüche, **dadurch gekennzeichnet,**
**dass** das Kapselhüllen- oder Kapselmatrixmaterial der Innenkapseln mindestens ein pharmakologisch unbedenkliches Polymer umfasst oder hieraus besteht, insbesondere ausgewählt aus synthetischen, natürlichen oder naturidentischen Polymeren und Polymerisaten, bevorzugt ausgewählt aus der Gruppe von (A) Polysacchariden, insbesondere Alginaten, Cellulose und Cellulosederivaten, Chitosanen, Stärke und Stärkederivaten, Agar-Agar, Carrageenen, Pektinen, Galactomannanen, Guaranen, Dextranen, Xanthanen, Glucanen und Gummi arabicum; (B) Proteinen, insbesondere Gelatine und Caseinaten; (C) Polylactiden, insbesondere Homo- und Copolymeren der Milchsäure; (D) Aminoharzen; (E) Poly(meth)acrylaten; (F) Polyharnstoffen; (G) Polyelektrolyten oder Polyelektrolytkomplexen; (H) Wachsen; (I) Paraffinen; (J) Kolloiden und Hydrokolloiden, insbesondere auf Polysaccharid- und/oder Proteinbasis; sowie deren Mischungen und Kombinationen, besonders bevorzugt aus der Gruppe von (A) Polysacchariden, insbesondere Alginaten, Cellulose und Cellulosederivaten, Chitosanen, Stärke und Stärkederivaten, Agar-Agar, Carrageenen, Pektinen, Galactomannanen, Guaranen, Dextranen, Xanthanen, Glucanen und Gummi arabicum; (B) Proteinen, insbesondere Gelatine und Caseinaten; (C) Polylactiden, insbesondere Homo- und Copolymeren der Milchsäure; sowie deren Mischungen und Kombinationen.

4. Darreichungsform nach einem der vorangehenden Ansprüche, **dadurch gekennzeichnet, dass** die Innenkapseln mittels Vertropfungsverfahren, Grenzflächenpolykondensationsverfahren, Grenzflächenpolyadditionsverfahren, Lösemittelverdampfungsverfahren, Sprühtrocknungsverfahren oder Phasentrennungsverfahren, bevorzugt mittels Vertropfungsverfahren, erhältlich sind und/oder
dass der Durchmesser, insbesondere der mittlere Durchmesser, der Innenkapseln mindestens um eine Größenordnung kleiner als der Durchmesser, insbesondere der mittlere Durchmesser, der Außenkapsel ist.

5. Darreichungsform nach einem der vorangehenden Ansprüche, **dadurch gekennzeichnet,**
**dass** das Verhältnis des Durchmessers, insbesondere des mittleren Durchmessers, der Innenkapseln zum Durchmesser, insbesondere zum mittleren Durchmesser, der Außenkapsel mindestens 1 : 2, insbesondere mindestens 1 : 5, vorzugsweise mindestens 1 : 10, besonders bevorzugt mindestens 1 : 15, ganz besonders bevorzugt mindestens 1 : 20, beträgt und/oder
**dass** das Verhältnis des Durchmessers, insbesondere des mittleren Durchmessers, der Innenkapseln zum Durchmesser, insbesondere zum mittleren Durchmesser, der Außenkapsel bis zu 1 : 100.000, insbesondere bis zu 1 : 50.000, vorzugsweise bis zu 1 : 15.000, besonders bevorzugt bis zu 1 : 10.000, ganz besonders bevorzugt bis zu 1 : 5.000, beträgt und/oder
**dass** das Verhältnis des Durchmessers, insbesondere des mittleren Durchmessers, der Innenkapseln zum Durchmesser, insbesondere zum mittleren Durchmesser, der Außenkapsel im Bereich von 1 : 2 bis 1 : 100.000, insbesondere im Bereich von 1 : 5 bis 1 : 50.000, vorzugsweise im Bereich von 1 : 10 bis 1 : 15.000, besonders bevorzugt im Bereich von 1 : 15 bis 1 : 10.000, ganz besonders bevorzugt im Bereich von 1 : 20 bis 1 : 5.000, variiert.

6. Darreichungsform nach einem der vorangehenden Ansprüche, **dadurch gekennzeichnet, dass** der Durchmesser, insbesondere der mittlere Durchmesser, der Innenkapseln im Bereich von 0,1 µm bis 10 mm, insbesondere im Bereich von 1 µm bis 8 mm, vorzugsweise im Bereich von 5 µm bis 7 mm, besonders bevorzugt im Bereich von 10 µm bis 5 mm, ganz besonders bevorzugt im Bereich von 25 µm bis 4 mm, noch mehr bevorzugt im Bereich von 50 µm bis 3 mm, variiert und/oder
**dass** die Innenkapseln eine monomodale Körnung und/oder Größenverteilung aufweisen und/oder
**dass** der Durchmesser, insbesondere der mittlere Durchmesser, der Außenkapsel im Bereich von 50 µm bis 100 mm, insbesondere im Bereich von 100 µm bis 75 mm, vorzugsweise im Bereich von 250 µm bis 60 mm, besonders bevorzugt im Bereich von 500 µm bis 50 mm, ganz besonders bevorzugt im Bereich von 750 µm bis 30 mm, noch mehr bevorzugt im Bereich von 1.000 µm bis 25 mm, variiert und/oder
dass die Außenkapsel mindestens zwei Innenkapseln, insbesondere mindestens fünf Innenkapseln, vorzugsweise mindestens zehn Innenkapseln, besonders bevorzugt mindestens fünfzehn Innenkapseln, ganz besonders bevorzugt mindestens zwanzig Innenkapseln, enthält und/oder dass die Außenkapsel bis zu 10.000 Innenkapseln, insbesondere bis zu 5.000 Innenkapseln, vorzugsweise bis zu 1.000 Innenkapseln, besonders bevorzugt bis zu 500 Innenkapseln, ganz besonders bevorzugt bis zu 100 Innenkapseln, enthält.

7. Darreichungsform nach einem der vorangehenden Ansprüche, **dadurch gekennzeichnet, dass** die Darreichungsform und/oder die Innenkapseln 1,8-Cineol als alleinigen Wirkstoff enthält bzw. enthalten oder aber dass die Darreichungsform und/oder die Innenkapseln 1,8-Cineol zumindest mit mindestens einem weiteren Wirkstoff, bevorzugt ausgewählt aus Terpenen, enthält bzw. enthalten und
**dass** die Innenkapseln 1,8-Cineol zusammen mit mindestens einem mit 1,8-Cineol mischbaren und/oder hierin löslichen, insbesondere bei 20 °C und Atmosphärendruck flüssigen, physiologisch unbedenklichen Träger (Exzipienten) enthalten, wobei der Träger ausgewählt ist aus mittelkettigen Triglyceriden (*Medium Chain Triglycerides,* MCT), ganz besonders bevorzugt Triglyceriden mit C₆-C₁₂-Fettsäureresten, und/oder insbesondere wobei der Träger in einem 1,8-Cineol/Träger-Mengenverhältnis im Bereich von 1.000 : 1 bis 1 : 1.000, insbesondere 100 : 1 bis 1 : 100, vorzugsweise 50 : 1 bis 1 : 50, besonders bevorzugt 10 : 1 bis 1 : 10, ganz besonders bevorzugt 5 : 1 bis 1 : 2, noch mehr bevorzugt 3 : 1 bis 1 : 1, eingesetzt wird

8. Darreichungsform nach einem der vorangehenden Ansprüche, **dadurch gekennzeichnet,**
**dass** die Darreichungsform den Wirkstoff 1,8-Cineol in absoluten Mengen von 10 bis 1.000 mg, insbesondere 25 bis 750 mg, vorzugsweise 50 bis 500 mg, besonders bevorzugt 75 bis 300 mg, enthält und/oder dass die Darreichungsform den Wirkstoff 1,8-Cineol in relativen Mengen von 0,01 bis 99 Gew.-%, insbesondere 0,1 bis 95 Gew.-%, vorzugsweise 1 bis 90 Gew.-%, besonders bevorzugt 5 bis 80 Gew.-%, enthält und/oder
**dass** die Darreichungsform in magensaftresistenter, dünndarmlöslicher Form vorliegt und/oder dass die Darreichungsform magensaftresistent, aber dünndarmlöslich ausgebildet ist, insbesondere wobei die Außenkapsel und/oder die Innenkapseln in magensaftresistenter, dünndarmlöslicher Form vorliegt bzw. vorliegen, bevorzugt mit einer magensaftresistenten, dünndarmlöslichen Beschichtung oder Hülle versehen ist bzw. sind.

9. Verfahren zur Herstellung einer cineolhaltigen Darreichungsform nach einem der vorangehenden Ansprüche,
wobei zunächst Mikrokapseln hergestellt werden, welche jeweils den Wirkstoff 1,8-Cineol zusammen mit mindestens einem mit 1,8-Cineol mischbaren und/oder hierin löslichen, bei 20 °C und Atmosphärendruck flüssigen, physiologisch unbedenklichen Träger (Exzipienten) enthalten, wobei der Träger ausgewählt ist aus der Gruppe von Triglyceriden, und
wobei nachfolgend eine Mehrzahl der zuvor hergestellten Mikrokapseln von einer äußeren Kapselhülle vollständig umgeben und zu einem Kapseln-in-Kapsel-System mit den Mikrokapseln als innere Kapseln (Innenkapseln) zusammengeführt und/oder zusammengefasst wird.

10. Verfahren nach Anspruch 9, **dadurch gekennzeichnet,**
**dass** die Herstellung der Mikrokapseln mittels Vertropfungsverfahren, Grenzflächenpolykondensationsverfahren, Grenzflächenpolyadditionsverfahren, Lösemittelverdampfungsverfahren, Sprühtrocknungsverfahren oder Phasentrennungsverfahren, bevorzugt mittels Vertropfungsverfahren, durchgeführt wird und/oder
**dass** die Mikrokapseln mittels eines Vertropfungsverfahrens hergestellt werden, wobei ein mikrokapselbildendes Ausgangsmaterial in Gegenwart von 1,8-Cineol und dem Trägers (Exzipienten) vertropft und zu 1,8-Cineol enthaltenden Mikrokapseln verfestigt wird.

11. Darreichungsform nach einem der Ansprüche 1 bis 8 zur Verwendung bei der prophylaktischen und/oder therapeutischen Behandlung von entzündlichen, infektexazerbierten oder allergischen Erkrankungen des menschlichen oder tierischen Körpers.

12. Darreichungsform nach Anspruch 11,
wobei die entzündlichen, infektexazerbierten oder allergischen Erkrankungen des menschlichen oder tierischen Körpers ausgewählt sein können aus der Gruppe von Autoimmunerkrankungen; Erkältungserkrankungen und grippalen Infekten und hiermit zusammenhängenden Erkrankungen und Infekten, insbesondere Infekten der oberen und unteren Atemwege, insbesondere Rhinitis, Sinusitis und bronchopulmonalen Erkrankungen; Atemwegserkrankungen, insbesondere bronchopulmonalen Erkrankungen, insbesondere Bronchitis, Asthma bronchiale und chronischen obstruktiven Lungenerkrankungen (COPD); entzündlichen Erkrankungen der Gallenwege, insbesondere Cholezystitis und Cholangitis; entzündlichen Erkrankungen der ableitenden Harnwege, insbesondere Glomerulonephritis, Pyelonephritis, Zystitis und Uritritis; entzündlichen Erkrankungen des Darms, insbesondere Morbus Crohn und Colitis ulcerosa; entzündlichen oder allergischen Hauterkrankungen, insbesondere Ekzemen und Dermatitis; rheumatoiden Erkrankungen, insbesondere Rheuma und/oder Rheumatismus sowie Erkrankungen des rheumatischen Formenkreises; systemisch kortikosteroidpflichtigen Erkrankungen, insbesondere Erkrankungen, welche mit systemisch verabreichten Kortikosteroiden therapiert werden.

13. Darreichungsform nach Anspruch 11 oder 12,
wobei das 1,8-Cineol in Tagesdosen von 50 bis 3.000 mg/diem, insbesondere 100 bis 2.000 mg/diem, vorzugsweise 200 bis 1.000 mg/diem, verabreicht wird und/oder insbesondere wobei das 1,8-Cineol zur Verabreichung in einer Tagesdosis von 50 bis 3.000 mg/diem, insbesondere 100 bis 2.000 mg/diem, vorzugsweise 200 bis 1.000 mg/diem, hergerichtet ist.

14. Arzneimittel oder Medizinprodukt, umfassend eine cineolhaltige Darreichungsform nach einem der vorangehenden Ansprüche.

15. Arzneimittel oder Medizinprodukt nach Anspruch 14, wobei das Arzneimittel oder Medizinprodukt für eine Verabreichung von 1,8-Cineol in einer Tagesdosis von 50 bis 3.000 mg/diem, insbesondere 100 bis 2.000 mg/diem, vorzugsweise 200 bis 1.000 mg/diem, hergerichtet ist.

## Claims

1. A dosage form containing cineole for the peroral application in the form of a capsule,
wherein the dosage form is embodied as a capsule-in-capsule system, wherein the capsule-in-capsule system has an outer capsule (exterior capsule) with an outer capsule shell, and a plurality of inner capsules (interior capsules) located inside of the outer capsule, wherein the inner capsules are completely surrounded by the capsule shell of the outer capsule, and the inner capsules are embodied as micro-capsules, each containing the active ingredient 1,8-cineole, and
wherein the inner capsules comprise 1,8-cineaole, together with at least one physiologically acceptable carrier (excipient) that is miscible with 1,8-cineole, or soluble therein, and is liquid at 20°C and at atmospheric pressure, wherein the carrier is selected from the group of triglycerides.

2. The dosage form according to claim 1, **characterized in that**
the inner capsules are present in the form of core/shell capsules, and/or
the inner capsules have a capsule shell or a capsule matrix, which completely encloses the active ingredient, and/or
the capsule shell or the capsule matrix material of the inner capsules may be identical or different with regard to the capsule shell material of the exterior capsule, and is preferably different.

3. The dosage form according to one of the preceding claims, **characterized in that** the capsule shell or capsule matrix material of the interior capsules comprises at least one pharmacologically acceptable polymer, or consists of the same, that is in particular selected from synthetic, natural, or nature-identical polymers and polymer sates, preferably selected from the group of (A) polysaccharides, in particular alginates, cellulose, and cellulose derivatives, chitosan's, starch, and starch derivatives, agar-agar, carrageen's, pectin's, galactomannans, Guarani, dextran's, xanthan's, gleans, and rubber Arabica; (B) proteins, in particular gelatin and castigates; (C) polylactides, in particular the homo and copolymers of lactic acid; (D) amino resins; (E) poly(meth)acrylates; (F) polyureas; (G) polyelectrolytes, or polyelectrolyte complexes; (H) wax; (I) paraffins; (J) colloids and hydrocolloids, in particular based on polysaccharide and/or protein; as well as the mixtures and combinations thereof, in particular preferably selected from the group of (A) polysaccharides, in particular alginates, cellulose, and cellulose derivatives, chitosan's, starch, and starch derivatives, agar-agar, carrageens, pectins, galactomannans, guarans, dextrans, xanthans, glucans, and rubber arabicum; (B) proteins, in particular gelatin and caseinates; (C) polylactides, in particular the homo and copolymers of lactic acid; as well as the mixtures and combinations thereof.

4. The dosage form according to one of the preceding claims, **characterized in that** the interior capsules are available by means of droplet forming methods, interfacial polycondensation methods, interfacial polyaddition methods, solvent evaporation methods, spray drying methods, or phase separation methods, preferably by means of droplet forming methods, and/or
the diameter, in particular the average diameter of the interior capsules is smaller by at least one order of magnitude than the diameter, in particular than the average diameter, of the exterior capsule.

5. The dosage form according to one of the preceding claims, **characterized in that** the ratio of the diameter, in particular of the average diameter, of the interior capsules to the diameter, in particular the average diameter, of the exterior capsule is at least 1:2, in particular at least 1:5, preferably at least 1:10, particularly preferred at least 1:15, most particularly preferred at least 1:20, and/or
the ratio of the diameter, in particular of the average diameter, of the interior capsules to the diameter, in particular the average diameter, of the exterior capsule is up to 1:100,000, in particular up to 1:50,000, preferably up to 1:15,000, particularly preferred up to 1:10,000, most particularly preferred up to 1:5,000, and/or
the ratio of the diameter, in particular of the average diameter, of the interior capsules to the diameter, in particular the average diameter, of the exterior capsule varies within the range of 1:2 to 1:100,000, in particular in the range of 1:5 to 1:50,000, preferably in the range of 1:10 to 1:15,000, particularly preferred in the range of 1:15 to 1:10,000, most particularly preferred in the range of 1:20 to 1:5,000.

6. The dosage form according to one of the preceding claims, **characterized in that** the diameter, in particular of the average diameter, of the interior capsules varies within the range of 0.1 µm to 10 mm, in particular within the range of 1 µm to 8 mm, preferably within the range of 5 µm to 7 mm, particularly preferred within the range of 10 µm to 5 mm, most particularly preferred within the range of 25 µm to 4 mm, still more preferred within the range of 50 µm to 3 mm, and/or
the interior capsules have a monomodal grain size, and/or size distribution, and/or
the diameter, in particular of the average diameter, of the exterior capsule varies within the range of 50 µm to 100 mm, in particular within the range of 100 µm to 75 mm, preferably within the range of 250 µm to 60 mm, particularly preferred within the range of 500 µm to 50 mm, most particularly preferred within the range of 750 µm to 30 mm, still more preferred within the range of 1,000 µm to 25 mm, and/or
the exterior capsule contains at least two interior capsules, in particular at least five interior capsules, preferably at least ten interior capsules, particularly preferred at least fifteen interior capsules, most particularly preferred at least twenty interior capsules, and/or the exterior capsule contains up to 10,000 interior capsules, in particular up to 5,000 interior capsules, preferably up to 1,000 interior capsules, particularly preferred at least up to 500 interior capsules, most particularly preferred up to 100 interior capsules.

7. The dosage form according to one of the preceding claims, **characterized in that** the dosage form and/or the interior capsules contains/contain 1,8-cineole as the single active ingredient, or the dosage form and/or the interior capsules contains/contain 1,8-cineole together with at least one additional active ingredient, which is preferably selected from terpenes, and
the interior capsules contain 1,8-cineole together with at least one physiologically acceptable carrier (excipient) that is miscible with 1,8-cineole, or soluble therein, and is liquid at 20°C and at atmospheric pressure, wherein the carrier is selected from medium-chain triglycerides (MCT), most particularly preferred from triglycerides having C₆-C₁₂ fatty acid residues, and/or in particular, wherein the carrier is utilized at a 1,8-cineole/carrier proportion within the range of 1,000:1 to 1:1,000, in particular 100:1 to 1:100, preferably 50:1 to 1:50, particularly preferred 10:1 to 1:10, most particularly preferred 5:1 to 1:2, still more preferred 3:1 to 1:1.

8. The dosage form according to one of the preceding claims, **characterized in that** the dosage form contains the active ingredient 1,8-cineaole in absolute amounts from 10 to 1,000 mg, in particular 25 to 750 mg, preferably 50 to 500 mg, particularly preferred 75 to 300 mg, and/or the dosage form contains the active ingredient 1,8-cineole in relative amounts from 0.01 to 99% by weight, in particular 0.1 to 95% by weight, preferably 1 to 90% by weight, particularly preferred 5 to 80% by weight, and/or
the dosage form is present in enteric-resistant, small intestine-soluble form, and/or the dosage form is embodied in an enteric-resistant, but small intestine-soluble manner, in particular, wherein the exterior capsule and/or the interior capsules is/are present in enteric-resistant, small intestine-soluble form, preferably being equipped with an enteric-resistant, small intestine-soluble coating or shell.

9. A method for the production of a dosage form containing cineole according to one of the preceding claims,
wherein initially microcapsules are produced, each comprising the active ingredient 1,8-cineole, together with at least one physiologically acceptable carrier (excipient) that is miscible with 1,8-cineole, or soluble therein, and is liquid at 20°C and at atmospheric pressure, wherein the carrier is selected from the group of triglycerides, and
wherein subsequently a plurality of previously produced microcapsules is completely surrounded by an outer capsule shell, and is combined and/or joined into a capsule-in-capsule system, with the microcapsules being the inner capsules (interior capsules).

10. The method according to claim 9, **characterized in that**
the production of the microcapsules is carried out by means of droplet forming methods, interfacial polycondensation methods, interfacial polyaddition methods, solvent evaporation methods, spray drying methods, or phase separation methods, preferably by means of droplet forming methods, and/or
the microcapsules are produced by means of a droplet forming method, wherein a microcapsule forming base material is provided in droplets in the presence of 1,8-cineole and the carrier (excipient), and is solidified into microcapsules containing 1,8-cineole.

11. The dosage form according to one of the claims 1 to 8 for use in the prophylactic and/or therapeutic treatment of inflammatory, infection exacerbating, or allergenic diseases of the human or animal body.

12. The dosage form according to claim 11,
wherein the inflammatory, infection exacerbating, or allergenic diseases of the human or animal body may be selected from the group of autoimmune diseases; the common cold or the flu, as well as diseases and infections related to the same, in particular infections of the upper and lower respiratory systems, in particular rhinitis, sinusitis, and bronchopulmonary diseases; respiratory diseases, in particular bronchopulmonary diseases, in particular bronchitis, bronchial asthma, and chronic obstructive pulmonary diseases (COPD); inflammatory diseases of the biliary tract, in particular cholecystitis and cholangitis; inflammatory diseases of the urinary tract collection system, in particular glomerulonephritis, pyelonephritis, cystitis, and uritritis; inflammatory diseases of the colon, in particular Crohn's disease and colitis ulcerosa; inflammatory or allergenic skin disease, in particular eczemas and dermatitis; rheumatoid diseases, in particular rheumatism, as well as diseases of the rheumatoid morphogenetic region; systemic corticosteroid-obligatory diseases, in particular diseases, which are treated with systemically administered corticosteroids.

13. The dosage form according to claims 11 or 12,
wherein the 1,8-cineole is administered in daily dosages of 50 to 3,000 mg/diem, in particular 100 to 2,000 mg/diem, preferably 200 to 1,000 mg/diem, and/or in particular, wherein the 1,8-cineole is provided for the administration in a daily dosage of 50 to 3,000 mg/diem, in particular 100 to 2,000 mg/diem, preferably 200 to 1,000 mg/diem.

14. A pharmaceutical or medical product, comprising a dosage form containing cineole according to one of the preceding claims.

15. The pharmaceutical or medical product according to claim 14, wherein the pharmaceutical or the medical product is provided for the administration of 1,8-cineole in a daily dosage of 50 to 3,000 mg/diem, in particular 100 to 2,000 mg/diem, preferably 200 to 1,000 mg/diem.

## Revendications

1. Forme pharmaceutique contenant du cinéol pour administration par voie orale sous forme d'une gélule,
la forme pharmaceutique étant configurée sous forme d'un système gélules-dans-gélule, le système gélules-dans-gélule comportant une gélule extérieure (gélule extérieure) comportant une enveloppe de gélule extérieure, et un grand nombre de gélules intérieures (gélules intérieures) se trouvant dans la gélule extérieure, les gélules intérieures étant entièrement entourées par l'enveloppe de gélule de la gélule extérieure, et les gélules intérieures étant configurées comme des microgélules dont chacune contient le principe actif 1,8-cinéol, et
dans laquelle les gélules intérieures contiennent du 1,8-cinéol, en même temps qu'au moins un support (excipient) physiologiquement inoffensif, liquide à 20°C et sous la pression atmosphérique, miscible au 1,8-cinéol et/ou soluble dans ce dernier, le support étant choisi dans le groupe des triglycérides.

2. Forme pharmaceutique selon la revendication 1, **caractérisée en ce que**
les gélules intérieures se présentent sous forme de gélules coeur/enveloppe, et/ou
les gélules intérieures comprennent une enveloppe de gélule ou une matrice de gélule, qui entoure complètement le principe actif, et/ou
le matériau de l'enveloppe de gélule ou de la matrice de gélule des gélules intérieures pouvant être identique au matériau de l'enveloppe de gélule de la gélule extérieure, ou en être différent, et de préférence en est différent.

3. Forme pharmaceutique selon l'une des revendications précédentes, **caractérisée en ce que**
le matériau de l'enveloppe de gélule ou de la matrice de gélule des gélules intérieures comprend au moins un polymère pharmacologiquement inoffensif ou en est constitué, en particulier choisi parmi les polymères et les polymérisats synthétiques, naturels ou identiques à la nature, de préférence choisis dans le groupe (A) des polysaccharides, en particulier des alginates, de la cellulose et des dérivés de la cellulose, des chitosanes, de l'amidon et des dérivés de l'amidon, de l'agar-agar, des carragènes, des pectines, des galactomannanes, des guaranes, des dextrans, des xanthanes, des glucanes et de la gomme arabique ; (B) des protéines, en particulier de la gélatine et des caséinates ; (C) des polylactides, en particulier des homo- et copolymères de l'acide lactique ; (D) des résines aminées ; (E) des poly(méth)acrylates ; (F) des polyurées ; (G) des polyélectrolytes et des complexes polyélectrolytiques ; (H) des cires ; (I) des paraffines ; (J) des colloïdes et des hydrocolloïdes, en particulier à base de polysaccharides et/ou de protéines ; ainsi que de leurs mélanges et combinaisons, d'une manière particulièrement préférée dans le groupe (A) des polysaccharides, en particulier des alginates, de la cellulose et des dérivés de la cellulose, des chitosanes, de l'amidon et des dérivés de l'amidon, de l'agar-agar, des carragènes, des pectines, des galactomannanes, des guaranes, des dextrans, des xanthanes, des glycanes et de la gomme arabique ; (B) des protéines, en particulier de la gélatine et des caséinates ; (C) des polylactides, en particulier des homo- et copolymères de l'acide lactique ; ainsi que leurs mélanges et combinaisons.

4. Forme pharmaceutique selon l'une des revendications précédentes, **caractérisée en ce que** les gélules internes peuvent être obtenues par des procédés de formation de gouttes, des procédés de polycondensation interfaciale, des procédés de polyaddition interfaciale, des procédés par évaporation de solvant, des procédés de séchage par atomisation ou des procédés de séparation de phases, de préférence par des procédés de formation de gouttes, et/ou
que le diamètre, en particulier le diamètre moyen, des gélules intérieures est au moins d'un ordre de grandeur inférieur au diamètre, en particulier le diamètre moyen, de la gélule extérieure.

5. Forme pharmaceutique selon l'une des revendications précédentes, **caractérisée en ce que**
le rapport du diamètre, en particulier du diamètre moyen, des gélules intérieures au diamètre, en particulier au diamètre moyen, de la gélule extérieure, est d'au moins 1:2, en particulier d'au moins 1:5, de préférence d'au moins 1:10, d'une manière particulièrement préférée d'au moins 1:15, d'une manière tout particulièrement préférée d'au moins 1:20, et/ou
le rapport du diamètre, en particulier du diamètre moyen, des gélules intérieures au diamètre, en particulier au diamètre moyen, de la gélule extérieure, peut aller jusqu'à 1:100 000, en particulier jusqu'à 1:50 000, de préférence jusqu'à 1:15 000, d'une manière particulièrement préférée jusqu'à 1:10 000, d'une manière tout particulièrement préférée jusqu'à 1:5 000 ; et/ou
le rapport du diamètre, en particulier du diamètre moyen, des gélules intérieures au diamètre, en particulier le diamètre moyen, de la gélule extérieure, varie dans la plage de 1:2 à 1:100 000, en particulier dans la plage de 1:5 à 1:50 000, de préférence dans la plage de 1:10 à 1:15 000, d'une manière particulièrement préférée dans la plage de 1:15 à 1:10 000, d'une manière tout particulièrement préférée dans la plage de 1:20 à 1:5 000.

6. Forme pharmaceutique selon l'une des revendications précédentes, **caractérisée en ce que**
le diamètre, en particulier le diamètre moyen, des gélules intérieures varie dans la plage de 0,1 µm à 10 mm, en particulier dans la plage de 1 µm à 8 mm, de préférence dans la plage de 5 µm à 7 mm, d'une manière particulièrement préférée dans la plage de 10 µm à 5 mm, d'une manière tout particulièrement préférée dans la plage de 25 µm à 4 mm, d'une manière encore plus préférée dans la plage de 50 µm à 3 mm, et/ou
les gélules intérieures présentent une granulométrie et/ou une distribution granulométrique monomodale, et/ou
le diamètre, en particulier le diamètre moyen, de la gélule extérieure varie dans la plage de 50 µm à 100 mm, en particulier dans la plage de 100 µm à 75 mm, de préférence dans la plage de 250 µm à 60 mm, d'une manière particulièrement préférée dans la plage de 500 µm à 50 mm, d'une manière tout particulièrement préférée dans la plage de 750 µm à 30 mm, d'une manière encore plus préférée dans la plage de 1 000 µm à 25 mm, et/ou
la gélule extérieure contient au moins deux gélules intérieures, en particulier au moins cinq gélules intérieures, de préférence au moins dix gélules intérieures, d'une manière particulièrement préférée au moins quinze gélules intérieures, d'une manière tout particulièrement préférée au moins vingt gélules intérieures, et la gélule extérieure contient jusqu'à 10 000 gélules intérieures, en particulier jusqu'à 5 000 gélules intérieures, de préférence jusqu'à 1 000 gélules intérieures, d'une manière particulièrement préférée jusqu'à 500 gélules intérieures, d'une manière tout particulièrement préférée jusqu'à 100 gélules intérieures.

7. Forme pharmaceutique selon l'une des revendications précédentes, **caractérisée en ce que**
la forme pharmaceutique et/ou les gélules intérieures contiennent le 1,8-cinéol en tant que principe actif unique, ou cependant que la forme pharmaceutique et/ou les gélules antérieures contiennent du 1,8-cinéol au moins en même temps qu'au moins un principe actif, de préférence choisi parmi les terpènes, et
les gélules intérieures contiennent du 1,8-cinéol en même temps qu'au moins un support (excipient) physiologiquement inoffensif, en particulier liquide à 20°C et sous la pression atmosphérique, miscible au 1,8-cinéol et/ou soluble dans ce dernier, le support étant choisi parmi les triglycérides à chaîne moyenne (Medium Chain Triglycerides, MCT), d'une manière tout particulièrement préférée parmi les triglycérides comportant des résidus acides gras en C₆-C₁₂, et/ou en particulier où le support est utilisé selon un rapport en poids 1,8-cinéol/support compris dans la plage de 1 000:1 à 1:1 000, en particulier de 100:1 à 1:100, de préférence de 50:1 à 1:50, d'une manière particulièrement préférée de 10:1 à 1:10, d'une manière tout particulièrement préférée de 5:1 à 1:2, d'une manière encore plus préférée de 3:1 à 1:1.

8. Forme pharmaceutique selon l'une des revendications précédentes, **caractérisée en ce que**,
la forme pharmaceutique contient le principe actif 1,8-cinéol en des quantités absolues de 10 à 1 000 mg, en particulier de 25 à 750 mg, de préférence de 50 à 500 mg, d'une manière particulièrement préférée de 75 à 300 mg, et/ou que la forme pharmaceutique contient le principe actif 1,8-cinéol en des quantités relatives de 0,01 à 99 % en poids, en particulier de 0,1 à 95 % en poids, de préférence de 1 à 90 % en poids, d'une manière particulièrement préférée de 5 à 80 % en poids, et/ou
la forme pharmaceutique se présente sous une forme gastro-résistante, soluble dans l'intestin grêle, et/ou que la forme pharmaceutique est configurée de façon à être gastro-résistante, mais soluble dans l'intestin grêle, en particulier où la gélule extérieure et/ou les gélules intérieures se présentent sous une forme gastro-résistante, soluble dans l'intestin grêle, et sont pourvues d'un enrobage ou d'une enveloppe gastro-résistante, soluble dans l'intestin grêle.

9. Procédé de fabrication d'une forme pharmaceutique contenant du cinéol selon l'une des revendications précédentes,
dans lequel sont fabriquées au moins des microgélules, dont chacune contient le principe actif 1,8-cinéol en même temps qu'au moins un support (excipient) physiologiquement inoffensif, liquide à 20°C et sous la pression atmosphérique, miscible au 1,8-cinéol et/ou soluble dans ce dernier, le support étant choisi dans le groupe des triglycérides, et/ou
dans lequel un grand nombre de microgélules fabriquées au préalable sont ensuite entièrement entourées d'une enveloppe de gélule extérieure, et réunies et/ou regroupées en un système gélules-dans-gélule, avec les microgélules en tant que gélules intérieures (gélules intérieures).

10. Procédé selon la revendication 9, **caractérisé en ce que**
la fabrication des microgélules est mise en oeuvre par des procédés de formation de gouttes, des procédés de polycondensation interfaciale, des procédés de polyaddition interfaciale, des procédés par évaporation de solvant, des procédés de séchage par atomisation ou des procédés par séparation de phases, de préférence par des procédés de formation de gouttes, et/ou
les microgélules sont fabriquées par un procédé de formation de gouttes, dans lequel une matière de départ génératrice de microgélules est transformée en gouttes en présence de 1,8-cinéol et du support (excipient) et est solidifiée pour donner des microgélules contenant le 1,8-cinéol.

11. Forme pharmaceutique selon l'une des revendications 1 à 8 pour utilisation lors du traitement prophylactique et/ou thérapeutique de maladies inflammatoires, exacerbées par une infection ou allergiques, du corps humain ou animal.

12. Forme pharmaceutique selon la revendication 11, pour laquelle les maladies inflammatoires, exacerbées par une infection ou allergique du corps humain ou animal peuvent être choisies dans le groupe consistant en les maladies auto-immunes, les maladies de type rhume et les infections grippales, et les maladies et infections qui s'y rattachent, en particulier les infections des voies respiratoires supérieures et inférieures, notamment la rhinite, la sinusite et les maladies bronchopulmonaires ; les maladies des voies respiratoires, en particulier les maladies bronchopulmonaires, notamment la bronchite, l'asthme bronchique et la bronchopneumopathie chronique obstructive (COPD) ; les maladies inflammatoires des voies biliaires, en particulier la cholécystite et la cholongite ; les maladies inflammatoires des voies urinaires efférentes, en particulier la glomérulonéphrite, la pyélonéphrite, la cystite et l'uréthrite ; les maladies inflammatoires de l'intestin, en particulier la maladie de Crohn et la recto-colite hémorragique, les maladies inflammatoires ou allergiques de la peau, en particulier les eczémas et la dermatite ; les maladies rhumatismales, en particulier le rhume et/ou les maladies de type rhumatisme, ainsi que les maladies de la catégorie du rhumatisme ; les maladies exigeant des corticostéroïdes par voie systémique, en particulier les maladies qui sont traitées à l'aide de corticostéroïdes administrés par voie systémique.

13. Forme pharmaceutique selon la revendication 11 ou 12, dans laquelle le 1,8-cinéol est administré à des doses journalières de 50 à 3 000 mg/jour, en particulier de 100 à 2 000 mg/jour, de préférence de 200 à 1 000 mg/jour, et/ou en particulier dans laquelle le 1,8-cinéol est destiné à une administration à une dose journalière de 50 à 3 000 mg/jour, en particulier de 100 à 2 000 mg/jour, de préférence de 200 à 1 000 mg/jour.

14. Médicament ou produit médicinal, comprenant une forme pharmaceutique contenant du cinéol selon l'une des revendications précédentes.

15. Médicament ou produit médicinal selon la revendication 14, le médicament ou le produit médicinal étant destiné à une administration de 1,8-cinéol à une dose journalière de 50 à 3 000 mg/jour, en particulier de 100 à 2 000 mg/jour, de préférence de 200 à 1 000 mg/jour.
